Europäisches Patentamt

European Patent Office

Office européen des brevets

(19)

(11) Veröffentlichungsnummer: **0 103 723**
**B1**

(12) **EUROPÄISCHE PATENTSCHRIFT**

(45) Veröffentlichungstag der Patentschrift:
18.12.85

(21) Anmeldenummer: 83107680.7

(22) Anmeldetag: 04.08.83

(51) Int. Cl.[4]: **C 07 C 85/12**, C 07 C 87/14,
B 01 J 23/74

(54) Verfahren zur Herstellung von primären Aminen.

(30) Priorität: **21.08.82 DE 3231193**

(43) Veröffentlichungstag der Anmeldung:
**28.03.84 Patentblatt 84/13**

(45) Bekanntmachung des Hinweises auf die Patenterteilung:
**18.12.85 Patentblatt 85/51**

(84) Benannte Vertragsstaaten:
**BE DE FR GB IT NL**

(56) Entgegenhaltungen:
**DE - A - 2 304 264**
**DE - A - 2 429 293**
**FR - A - 2 055 075**
**FR - A - 2 322 127**

**C. DUVAL: Dictionnaire de la Chimie, 3ème ed. Seite 78**
**LANGE'S "HANDBOOK OF CHEMISTRY" Seite 4-63**

(73) Patentinhaber: **BASF Aktiengesellschaft,**
**Carl-Bosch-Strasse 38, D-6700 Ludwigshafen (DE)**

(72) Erfinder: **Frank, Gerhard, Dr., Heidelberger Strasse 11,**
**D-6945 Hirschberg (DE)**
Erfinder: **Rudolf, Peter, Dr., Wiesenstrasse 1,**
**D-6708 Neuhofen (DE)**
Erfinder: **Neubauer, Gerald, Dr., Mozartstrasse 24,**
**D-6940 Weinheim (DE)**
Erfinder: **Duffner, Paul, Dr., Bozener Strasse 14,**
**D-6700 Ludwigshafen (DE)**
Erfinder: **Ohlinger, Manfred, Dr.,**
**Anselm-Feuerbach-Strasse 13, D-6710 Frankenthal (DE)**
Erfinder: **Wilfinger, Hans Jeorg, Dr., Buschstrasse 11,**
**D-6707 Schifferstadt (DE)**
Erfinder: **Pfannebecker, Emil,**
**Robert-Schumann-Strasse 55a, D-6707 Schifferstadt**
**(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Bei der Herstellung von Aminen durch Hydrieren von Nitrilen, z.B. Hexamethylendiamin aus Adiponitril, werden Kobalt enthaltende Katalysatoren wegen ihrer hohen Selektivität bevorzugt verwendet. Solche Verfahren sind beispielsweise aus den DE-PSen 10 72 972 und 12 59 899 bekannt. Die Lebensdauer der verwendeten Kobaltkatalysatoren entspricht jedoch nicht mehr den technischen Anforderungen. Darüber hinaus weisen Befunde darauf hin, dass Stäube metallischen Kobalts und seiner schwerlöslichen Verbindungen aus arbeitshygienischen Gründen vermieden werden sollten. Es wurden auch schon Eisen enthaltende Katalysatoren für die Hydrierung von Nitrilen zu Aminen verwendet. Bei der Verwendung von Eisenkatalysatoren müssen jedoch höhere Temperaturen angewandt werden. Dies führt in verstärktem Masse zur Bildung von Nebenprodukten. Bei der Herstellung von Hexamethylendiamin bilden sich beispielsweise Azacycloheptan und schwer vom Hexamethylendiamin abtrennbare Diamine, wie 2-Aminomethylcyclopentylamin und 1,2-Diaminocyclohexan sowie bis-Hexamethylentriamin. So ist aus der DE-OS 24 29 293 bekannt, dass man durch Schmelzen von Magnetit und Reduktion mit Wasserstoff einen Katalysator erhält, der bei hot-spot-Temperaturen von 150 bis 170° C eine Selektivität von 98 bis 99% an Hexamethylendiamin erzielt. Die Gehalte an 1,2-Diaminocyclohexan liegen jedoch bei 0,2 Gew.%. Auch nach dem in der DE-AS 20 34 380 beschriebenen Verfahren, bei dem als Katalysator eine granulierte Eisenverbindung, die durch Reduktion mit Wasserstoff in metallisches Eisen überführt worden ist, verwendet wird, erzielt man nur Selektivitäten von 97 bis 98,8 Gew.%. Solche Eisenkatalysatoren erfüllen noch nicht alle Erwartungen, die in der Technik an sie gestellt werden, und sind deshalb verbesserungsbedürftig.

Es war deshalb die technische Aufgabe gestellt, Eisenkatalysatoren zur Verfügung zu stellen, die bei der Hydrierung von Nitrilen zu Aminen eine lange Lebensdauer haben, niedrigere Hydriertemperaturen erlauben, wenig Nebenprodukte erzeugen und eine hohe Selektivität aufweisen.

Diese technische Aufgabe wird gelöst in einem Verfahren zur Herstellung von gesättigten primären Aminen durch Umsetzen von Nitrilen mit Wasserstoff bei erhöhter Temperatur und unter erhöhtem Druck in Gegenwart von Ammoniak und metallischen Eisenkatalysatoren, die durch Reduktion von Eisenverbindungen mit Wasserstoff bei Temperaturen $\leqslant 500°$ C erhalten worden sind, dadurch gekennzeichnet, dass metallische Eisenkatalysatoren verwendet werden, die aus anisometrischen $\gamma$-Eisenoxidteilchen erhalten worden sind.

Das neue Verfahren hat den Vorteil, dass die verwendeten Katalysatoren eine lange Lebensdauer haben und sich auch nach längerem Gebrauch noch durch überlegene Eigenschaften auszeichnen. Das neue Verfahren hat ferner den Vorteil, dass bei niedrigeren Hydriertemperaturen eine hohe Selektivität erzielt wird. Darüber hinaus zeichnet sich das neue Verfahren dadurch aus, dass weniger Nebenprodukte anfallen, die schwer von den Wertprodukten abtrennbar sind.

Als Ausgangsstoffe verwendet man bevorzugt aliphatische, cycloaliphatische, araliphatische oder aromatische Nitrile, mit bis zu 20 Kohlenstoffatomen. Die Nitrilgruppe kann ein- oder mehrmals im Molekül vorkommen. Für die Hydrierung eignen sich gesättigte oder olefinisch ungesättigte Nitrile. Sie können auch unter Reaktionsbedingungen inerte Substituenten haben, wie über Etherbrücken gebundene Alkylreste mit 1 bis 4 Kohlenstoffatomen. Besonders bevorzugte Ausgangsstoffe sind Alkannitrile oder Alkandinitrile mit 3 bis 18 Kohlenstoffatomen. Geeignete Nitrile sind beispielsweise Propionitril, Acetonitril, Acrylnitril, Benzylcyanid, Benzonitril, Glutarsäuredinitril oder Adipinsäuredinitril.

Besondere technische Bedeutung hat das Verfahren bei der Hydrierung von Adipinsäuredinitril zu Hexamethylendiamin erlangt.

Die Umsetzung führt man im allgemeinen unter Drücken von 100 bis 400 bar, vorzugsweise 200 bis 300 bar, durch. Vorteilhaft hält man Temperaturen von 80 bis 140° C, vorzugsweise 100 bis 120° C, ein.

Die Umsetzung wird in Gegenwart von Ammoniak durchgeführt. Es hat sich bewährt, wenn das Volumenverhältnis von Nitril zu Ammoniak von 1:2 bis 1:20, vorzugsweise 1:6 bis 1:12, beträgt. Es ist auch möglich, Ammoniak teilweise durch zurückgeführtes rohes Hydriergemisch, das im wesentlichen aus Amin und Ammoniak besteht, zu ersetzen.

Erfindungsgemäss verwendet man Eisenkatalysatoren, die aus anisometrischen $\gamma$-Eisenoxidteilchen durch Reduktion mit Wasserstoff bei einer Temperatur $\leqslant 500°$ C erhalten worden sind. Vorteilhaft weisen die metallischen Eisenteilchen einen Reduktionsgrad $\geqslant 95\%$ auf. Unter Reduktionsgrad ist der Anteil des verfügbaren Eisens in % zu verstehen, der in metallischer Form vorliegt. Bevorzugt geht man von anisometrischem $\gamma$-Eisen-III-oxid und $\gamma$-Eisen-III-oxidhydrat aus. $\gamma$-Eisen-III-oxidhydrat, das unter dem Namen Lepidokrokit bekannt ist, wird bevorzugt verwendet. Es ist z.B. nach dem in der DE-AS 10 61 760 beschriebenen Verfahren erhältlich. Die anisometrischen Eisenoxide haben z.B. eine mittlere Teilchenlänge von 0,1 bis 2 mμ, vorzugsweise 0,2 bis 1,2 mμ, bei einem Längen-zu-Dickenverhältnis von 5:1 bis 40:1, einer spezifischen Oberfläche nach BET von 25 bis 80 m²/g. In gleicher Weise lassen sich auch die getemperten Produkte der genannten Eisen-III-oxide verwenden. Die Temperung erfolgt zweckmässig bei 250 bis 700° C. Vorteilhaft haben die verwendeten Eisenoxidteilchen einen Alkaligehalt von < 0,1 Gew.%, berechnet als Natriumoxid.

Die erfindungsgemässen metallischen Eisenkatalysatoren können als Suspension angewandt werden. Bevorzugt verwendet man jedoch geformte Eisenkatalysatormassen, die zusätzlich Gleitmittel, z.B. anorganische Stoffe mit Gitterstruktur, wie Talk oder insbesondere Graphit, enthalten. Vorteilhaft enthalten geformte Katalysatoren 1 bis 5 Gew.% Gleitmittel, bezogen auf die gesamte Katalysatormasse, aus Eisenteilchen und Gleitmittel. Besonders bewährt hat sich Graphit als Gleitmittel. Die geformte Eisenkatalysatormasse besteht somit im wesentlichen aus metallischen Eisenteilchen, geringen Mengen Eisenoxid entsprechend dem Reduktionsgrad der Eisenteilchen und einem Gleitmittel. Die geformte Eisenkatalysatormasse, z.B. in Form von Kugeln, Tabletten oder Strängen, weist vorteilhaft eine Druckhärte von $\geq$ 300 kp/cm² auf.

Die bevorzugt verwendeten geformten Eisenkatalysatormassen stellt man her, indem man von $\gamma$-Eisen-III-oxiden, insbesondere $\gamma$-Eisen-III-oxidhydrat (Lepidokrokit), ausgeht. In gleicher Weise lassen sich auch die getemperten Produkte der genannten $\gamma$-Eisen-III-oxide verwenden, wobei die Temperung zweckmässig bei 250 bis 700° C erfolgt. $\gamma$-Eisen-III-oxidhydrat erhält man beispielsweise aus wässerigen Lösungen von Eisensalzen durch Fällen mit Natronlauge nach einem Verfahren, wie es in der DE-AS 10 61 760 beschrieben wird. Vorteilhaft wäscht man die $\gamma$-Eisenoxidhydratpartikel, bis der Alkaligehalt < 0,1 Gew.%, berechnet als Natriumoxid, beträgt. Die nadelförmigen $\gamma$-Eisen-III-oxidteilchen werden mittels Wasserstoff in der Wirbelschicht, im Drehrohrofen oder vorzugsweise in einem gerührten Festbett bei einer Temperatur von 260 bis 500° C, insbesondere 300 bis 450° C, innerhalb von 3 bis 36 Stunden reduziert. Vorteilhaft wendet man einen trockenen Wasserstoffstrom an, wobei man eine relativ grosse Wasserstoffströmungsgeschwindigkeit einhält. Es hat sich bewährt, wenn man mindestens einen 60fachen Wasserstoffüberschuss anwendet. Vorteilhaft reduziert man so lange, bis der Reduktionsgrad $\geq$ 95% beträgt. So erhaltene nadelförmige, im wesentlichen aus Eisen bestehende Metallteilchen besitzen noch weitgehend die von den Ausgangsstoffen herrührende Gestalt und sind trotz der vorangegangenen Umwandlungsreaktion einheitlich.

Anschliessend werden die so erhaltenen Metallteilchen stabilisiert. Hierunter versteht man das Umhüllen der Metallteilchen mit einer Oxidschicht durch kontrollierte Oxidation, um die durch die grosse freie Oberfläche der kleinen Teilchen bedingte Pyrophorität zu beseitigen. Dies wird durch Überleiten eines Luft/Stickstoffgemisches unter genauer Einhaltung einer vorzugsweise 100° C, insbesondere 80° C, nicht übersteigenden Temperatur über das Metallpulver erreicht. Nach der Stabilisierung soll der Reduktionsgrad nicht unter 80, vorzugsweise nicht unter 90% liegen. Die stabilisierten Eisenteilchen haben eine Oberfläche nach BET von 4 bis 25 m²/g, vorzugsweise 8 bis 12 m²/g, sowie Partikellängen von 0,05 bis 2,0 m$\mu$, ferner Porenvolumina unterhalb 0,4 cm³/g, wobei das Verhältnis der Mikro- zu Makroporen zugunsten der Makroporen im Bereich von 1:6 bis 1:10 liegt.

Die so stabilisierten Eisenteilchen werden mit einem inerten Gleitmittel, vorzugsweise Graphit, vermischt. Vorteilhaft wendet man 2 bis 5 Gew.% Gleitmittel an. Das Gemisch aus stabilisierten Eisenteilchen und Gleitmittel wird zweckmässig unter Stickstoffabdeckung zu Formkörpern verarbeitet, z.B. zu Tabletten verpresst. Die Druckhärte der Formkörper soll $\geq$ 300 kp/cm² betragen. Die so erhaltenen Formkörper werden durch Behandeln mit Wasserstoff in relativ grossem Überschuss, z.B. dem 60fachen Überschuss, bei einer Temperatur $\leq$ 500° C, vorzugsweise 300 bis 460° C, bei Atmosphärendruck oder unter erhöhtem Druck, z.B. 100 bis 150 bar, aktiviert. Hierbei soll ein Reduktionsgrad von vorteilhaft $\geq$ 95% erreicht werden. Durch die Aktivierung steigt die Druckhärte der Formkörper z.B. von 300 auf 600 bis 800 kg/cm².

Die Hydrierung der Nitrile kann absatzweise erfolgen, vorteilhaft führt man die Umsetzung kontinuierlich durch, z.B. in Rieselfahrweise an fest angeordneten geformten Eisenkatalysatoren.

Die nach dem Verfahren der Erfindung erhältlichen Amine eignen sich zur Herstellung von Stabilisatoren. Hexamethylendiamin, das nach dem Verfahren der Erfindung erhalten wird, ist ein wichtiges Ausgangsprodukt zur Herstellung von Polyamid-6,6.

Das Verfahren nach der Erfindung sei an folgenden Beispielen veranschaulicht.

### Beispiel 1

Herstellung des Katalysators

600 kg nadelförmiger Lepidokrokit ($\gamma$-FeOOH) mit einem Chlorgehalt < 0,1% und einem Na$_2$O-Gehalt < 0,1%, hergestellt nach der DE-AS 10 61 760, mit einer spezifischen Oberfläche von 32 m²/kg, einer mittleren Nadellänge von 0,8 m$\mu$ und einem Längen:Dicken-Verhältnis der Nadeln von 22:1, einer Schüttdichte von 0,37 g/cm³ und mit einem pH-Wert von 7,2 werden in einem gerührten Festbett bei 400° C 38 h mit 400 Nm³/h Wasserstoff zu metallischem Eisen (Fe $\geq$ 95%) reduziert (stöchiometrischer Wasserstoffüberschuss: 64). Anschliessend wird in einem Stickstoffluftgemisch das pyrophore nadelige Metallpigment bei einer Temperatur von 60° C mit einer stabilisierenden Oxydschicht überzogen, wobei der Reduktionsgrad nicht unter 90% absinken soll. Die Ausbeute beträgt 400 kg. Die Sättigungsmagnetisierung der Eisenteilchen beträgt 153 nT m³/g in einem 160-kA/M-Messfeld. Die Eisenteilchen haben eine spezifische Oberfläche von 7,2 m²/g (nach der BET-Methode) und besitzen in der elektronenmikroskopischen Aufnahme eine anisotrope geometrische Form (Nadel- bzw. Stäbchenform).

Zur Herstellung von geformten Massen mit Durchmesser 5 mm und einer Höhe von 4 mm wird das stabilisierte pulverförmige Metallpigment mit 2 Gew.% Graphit vermischt und unter Stickstoffabdeckung tablettiert. Die Druckhärte der Tabletten soll nicht unter 300 kp/cm² liegen.

### Beispiel 2

In einem Reaktor der Länge 1 800 mm und einem inneren Durchmesser von 160 mm werden 350 l

der so hergestellten Formlinge eingefüllt und bei 360° C und 150 bar mit hohem Wasserstoffüberschuss über 24 h zum Zweck der Aufhebung der Passivierung behandelt. Der Wasserstoff wird dabei im Kreis über einen Kondensator zur Abscheidung des Reduktionswassers geführt.

Nach dem Abkühlen des Katalysators wird der Reaktor in Rieselfahrweise bei 270 bar Wasserstoffdruck stündlich mit einem Gemisch von 85 l Adipinsäuredinitril und 510 l flüssigem Ammoniak unter Kreisgasfahrweise des Wasserstoffs (400 Nm³/h) beschickt. Die Temperatur des Zulaufgemischs beträgt 78° C und die des Reaktorausgangs liegt bei 110° C; es entstehen hot-spot-Temperaturen von max. 119° C.

Die gaschromatographische Analyse des rohen Hexamethylendiamins nach Verdampfen des Ammoniaks aus dem Hydriergemisch ergibt 0,02 Gew.% Hexylamin, 0,09 Gew.% Azacycloheptan, 0,11 Gew.% 1,2-Diaminocyclohexan und 99,78 Gew.% Hexamethylendiamin sowie einen Aminocapronitrilgehalt ⩽ 0,01%. Der vorwiegend aus Bishexamethylentriamin bestehende Destillationsrückstand liegt bei 0,36%. Es errechnet sich eine Selektivität von 99,4% Hexamethylendiamin. Der Katalysator hatte nach einer Laufzeit von 300 Tagen unveränderte Aktivität und Selektivität ohne jegliche Regenerierung.

*Beispiel 3*

In dem unter Beispiel 2 beschriebenen Reaktor werden an dem nach Beispiel 1 hergestellten Katalysator stündlich 70 l Adipinsäuredinitril in 430 l flüssigem Ammoniak und 490 l rückgeführtem Hydriergemisch zu Hexamethylendiamin umgesetzt. Es wird ein Druck von 250 bar Wasserstoff und 350 Nm³/h Kreisgas eingehalten. Voller Umsatz des Adipinsäuredinitrils wird erreicht bei einer Zulauftemperatur von 77° C, dabei beträgt die Reaktorausgangstemperatur 104° C und $T_{max.}$ im Reaktor liegt bei 109° C.

Die gaschromatografische Analyse des rohen Hexamethylendiamins nach Verdampfen des Ammoniaks ergibt: 0,01% Hexylamin, 0,05% Azacycloheptan, 0,11% 1,2-Diaminocyclohexan, 0,002% 2-Aminomethylcyclopentylamin, 99,80% Hexamethylendiamin und 0,01% Aminocapronitril. Der Destillationsrückstand lag bei 0,40%, es errechnet sich eine Selektivität von 99,44% Hexamethylendiamin.

*Beispiel 4*

In einem Hochdruckreaktor von 2000 mm Länge und einem inneren Durchmesser von 45 mm werden 3 l des nach Beispiel 1 hergestellten Katalysators eingefüllt und die Passivierung des Katalysators nach Beispiel 2 aufgehoben. Pro h werden dem Reaktor 100 ml Adipinsäuredinitril und 1200 ml flüssiges Ammoniak zudosiert. Bei einer Hydriertemperatur von 109° C und einem Druck von 260 bar wird eine Selektivität von 99,63% Hexamethylendiamin erreicht. Das rohe Hexamethylendiamin enthält nur 0,04% Azacycloheptan und 0,09% 1,2-Diaminocyclohexan. Der Destillationsrückstand liegt bei 0,23%.

*Beispiel 5*

In einem 2-l-Schüttelautoklaven wurden 80 g 2-Methylglutarsäuredinitril und 100 ml fl. Ammoniak in Gegenwart von 80 g Katalysatortabletten, hergestellt gemäss Beispiel 1, bei 260 bar und 100° C bis zur Beendigung der Wasserstoffaufnahme hydriert. Es wurde bei vollständigem Umsatz des eingesetzten Dinitrils eine Selektivität von 98,8% 2-Methylpentamethylendiamin erzielt.

Analog wird unter den o.g. Hydrierbedingungen Propionitril in fl. Ammoniak mit einer Selektivität von 97,5% zu n-Propylamin hydriert.

*Beispiel 6 (Vergleich)*

Man verfährt wie in Beispiel 4 beschrieben, verwendet jedoch 3 l Katalysator, der durch Tablettierung eines Gemisches aus 98% γ-FeOOH und 2% Graphit hergestellt wurde. Der Katalysator wurde mittels Wasserstoff bei 450° C über 72 h drucklos reduziert. Es wurde ein Reduktionsgrad von 95% erreicht. Für vollen Umsatz von 400 g Adipinsäuredinitril pro h (in 1460 g $NH_3$/h) war eine Hydriertemperatur von 155° C bei 260 bar erforderlich (Rieselfahrweise). Die Selektivität des Katalysators lag bei 97,15% Hexamethylendiamin. Das rohe Hexamethylendiamin enthielt 1,56% zu weit umgewandelte Produkte und 1,29% cyclische Produkte (1,2-Diaminocyclohexan und Azacycloheptan).

*Beispiel 7 (Vergleich)*

Man verfährt wie in Beispiel 4 beschrieben, verwendet jedoch 3 l eines Katalysators, der durch Reduktion von α-FeOOH und Tablettierung des hierbei entstehenden Eisenmetallpigments nach oberflächlicher Passivierung und Vermischen mit 2% Graphit hergestellt wurde. Der Katalysator enthält durch die Fällung des α-FeOOH im alkalischen Bereich 0,18% Natriumhydroxid eingeschlossen. Im Reaktor wird die Passivierung durch 24stündige Behandlung mit drucklosem Wasserstoff bei 360° C aufgehoben. In Rieselfahrweise werden dann pro h 400 g Adipinsäuredinitril und 1460 g $NH_3$ zudosiert.

Voller Umsatz des Adipinsäuredinitrils wird bei 172° C erzielt. Die Selektivität liegt bei 97,8% Hexamethylendiamin, wobei 1,3% cyclische Nebenprodukte und 0,8% zu weit umgewandelte Produkte (vorwiegend Bishexamethylendiamin) anfallen.

**Patentansprüche**

1. Verfahren zur Herstellung von gesättigten primären Aminen durch Umsetzen von Nitrilen mit Wasserstoff bei erhöhter Temperatur und unter erhöhtem Druck in Gegenwart von Ammoniak und metallischen Eisenkatalysatoren, die durch Reduktion von Eisenverbindungen mit Wasserstoff bei Temperaturen ⩽ 500° C erhalten worden sind, dadurch gekennzeichnet, dass man metallische Eisenkatalysatoren verwendet, die aus anisometrischen γ-Eisenoxidteilchen erhalten worden sind.

2. Verfahren nach Anspruch 1, dadurch gekennzeichnet, dass die verwendeten Eisenoxidteilchen weniger als 0,1 Gew.% Alkali, berechnet als Natriumoxid, enthalten.

3. Verfahren nach den Ansprüchen 1 und 2, dadurch gekennzeichnet, dass man von anisometrischem γ-Eisen-III-oxidhydrat ausgeht.

4. Verfahren nach den Ansprüchen 1 bis 3, dadurch gekennzeichnet, dass man geformte Eisenkatalysatormassen verwendet, die durch Reduktion von anisometrischen γ-Eisenoxidteilchen bei einer Temperatur von 250 bis 500° C mit Wasserstoff zu metallischen Eisenteilchen, Stabilisieren der so erhaltenen metallischen Eisenteilchen durch Behandeln mit einem Gemisch aus Stickstoff und Luft, Verpressen der stabilisierten Eisenteilchen mit Gleitmitteln zu Formkörpern und Aktivieren der Formkörper durch Behandeln mit Wasserstoff bei einer Temperatur von ≤ 500° C erhalten worden sind.

5. Verfahren nach den Ansprüchen 1 bis 4, dadurch gekennzeichnet, dass die geformte Eisenkatalysatormasse 1 bis 5 Gew.% Graphit als Gleitmittel enthält.

6. Verfahren nach den Ansprüchen 1 bis 5, dadurch gekennzeichnet, dass die Formkörper eine Druckhärte von über 300 kp/cm² haben.

7. Verfahren nach den Ansprüchen 1 bis 6, dadurch gekennzeichnet, dass man die anisometrischen γ-Eisenoxidteilchen bis zu einem Reduktionsgrad von ≥ 95% reduziert.

8. Verfahren nach den Ansprüchen 1 bis 7, dadurch gekennzeichnet, dass man Alkannitrile oder Alkandinitrile mit 3 bis 18 Kohlenstoffatomen als Ausgangsstoffe verwendet.

## Claims

1. A process for the preparation of a saturated primary amine by reacting a nitrile with hydrogen at elevated temperature under superatmospheric pressure in the presence of ammonia and metallic iron catalyst which has been obtained by reducing an iron compound with hydrogen at ≤ 500° C, wherein a metallic iron catalyst which has been obtained from anisometric γ-iron oxide particles is used.

2. A process as claimed in claim 1, wherein the iron oxide particles used contain less than 0.1% by weight of alkali, calculated as sodium oxide.

3. A process as claimed in claims 1 and 2, wherein anisometric γ-iron-III oxide hydroxide is used as the starting substance.

4. A process as claimed in claims 1 to 3, wherein molded iron catalyst materials which have been obtained by reducing anisometric γ-iron oxide particles to metallic iron particles with hydrogen at from 250 to 500° C, stabilizing the resulting metallic iron particles by treatment with a mixture of nitrogen and air, compressing the stabilized iron particles together with lubricants to form moldings, and activating the moldings by treatment with hydrogen at ≤ 500° C are used.

5. A process as claimed in claims 1 to 4, wherein the molded iron catalyst material contains from 1 to 5% by weight of graphite as lubricant.

6. A process as claimed in claims 1 to 5, wherein the moldings have a compressive strength of more than 300 kp/cm².

7. A process as claimed in claims 1 to 6, wherein the anisometric γ-iron oxide particles are reduced to a degree of reduction of ≥ 95%.

8. A process as claimed in claims 1 to 7, wherein an alkanenitrile or alkanedinitrile of 3 to 18 carbon, atoms is used as the starting substance.

## Revendications

1. Procédé de préparation d'amines primaires saturées par réaction de nitriles avec l'hydrogène, à température et pression accrues en présence d'ammoniac et de catalyseurs au fer métallique, préparés par la réduction de composés du fer par l'hydrogène à une température inférieure ou égal à 500° C, caractérisé en ce que l'on utilise des catalyseurs au fer métallique, préparés à partir de particules anisométriques d'oxyde de fer γ.

2. Procédé suivant la revendication 1, caractérisé en ce que les particules d'oxyde de fer possèdent une teneur en alcali, calculé comme oxyde de sodium, inférieure à 0,1% en poids.

3. Procédé suivant les revendications 1 et 2, caractérisé en ce que l'on utilise comme produit de départ de l'oxyde ferrique γ hydraté anisométrique.

4. Procédé suivant les revendications 1 à 3, caractérisé en ce que l'on emploie des produits catalytiques au fer façonnés, obtenus par une réduction de particules anisométriques d'oxyde de fer γ par l'hydrogène, entre 250 et 500° C, en particules de fer métallique, une stabilisation des particules de fer métallique ainsi obtenues par un traitement dans un mélange d'air et d'azote, un compactage des particules de fer stabilisées avec des lubrifiants én éléments façonnés et une activation de ces éléments façonnés par un traitement à l'hydrogène à une température inférieure ou égale à 500° C.

5. Procédé suivant les revendications 1 à 4, caractérisé en ce que le catalyseur au fer façonné comprend entre 1 et 5% en poids de graphite comme lubrifiant.

6. Procédé suivant les revendications 1 à 5, caractérisé en ce que les éléments façonnés possèdent une résistance à l'écrasement supérieure à 300 kp/cm².

7. Procédé suivant les revendications 1 à 6, caractérisé en ce que les particules anisométriques d'oxyde de fer γ sont réduites jusqu'à un degré de réduction égal ou supérieur à 95%.

8. Procédé suivant les revendications 1 à 7, caractérisé en ce que l'on emploie comme composés de départ des alcane-nitriles ou des alcane-dinitriles en $C_3$ à $C_{18}$.